# EUROPÄISCHE PATENTSCHRIFT

(11) **EP 3 071 961 B1**
(45) Veröffentlichungstag und Bekanntmachung des Hinweises auf die Patenterteilung: **30.12.2020**
(21) Anmeldenummer: 14812415.9
(22) Anmeldetag: 21.11.2014
(51) Int. Cl.: G01N 30/72, G01N 27/62, G01N 30/20, G01N 33/497

(54) **VERFAHREN ZUR MESSUNG DER MENSCHLICHEN AUSATEMLUFT**
METHOD FOR MEASURING HUMAN EXHALED AIR
PROCÉDÉ DE MESURE DE L'AIR EXPIRÉ CHEZ L'HOMME

(30) Priorität: 22.11.2013 DE 102013112921
(43) Veröffentlichungstag der Anmeldung: 28.09.2016
(73) Patentinhaber: IMSPEX DIAGNOSTICS Ltd, Rhondda Cynon Taff CF45 4SN (GB)
(72) Erfinder: SIELEMANN, Stefanie, 44143 Dortmund (DE); WORTELMANN, Thomas, 44141 Dortmund (DE)
(74) Vertreter: Meinke, Jochen
(86) Internationale Anmeldenummer: PCT/EP2014/075236
(87) Internationale Veröffentlichungsnummer: WO 2015/075168

(56) Entgegenhaltungen:
- EP-A2- 0 573 060
- WO-A2-03/064994
- DE-A1-102007 033 906
- DE-A1-102009 038 238
- US-A1- 2005 085 740
- RISBY T H ET AL: "Current status of clinical breath analysis", APPLIED PHYSICS B ; LASERS AND OPTICS, SPRINGER, BERLIN, DE, Bd. 85, Nr. 2-3, 31. Mai 2006 (2006-05-31) , Seiten 421-426, XP019442368, ISSN: 1432-0649, DOI: 10.1007/S00340-006-2280-4

## Beschreibung

Die Erfindung betrifft ein Verfahren zur Messung der menschlichen Ausatemluft mittels Gaschromatografie-Ionenmobilitätsspektrometrie, bei welchem eine Ausatemluftprobe über einen Probeneinlass und ein Mehrwegeventil in eine Probenschleife gelangt und nachfolgend mittels eines Trägergases aus der Probenschleife über das Mehrwegeventil durch eine gaschromatografische Säule in ein Ionenmobilitätsspektrometer gefördert und gemessen wird.

Die Detektion von flüchtigen organischen Verbindungen in der menschlichen Atemluft mit Hilfe unterschiedlicher analytischer Verfahren ist vielfach beschrieben. Auch Ionenmobilitätsspektrometer sowie deren Kopplungen mit gaschromatografischer Vortrennung sind in Forschungsprojekten bereits zum Einsatz gekommen.

So ist z.B. aus Journal of Chromatography A, 1084 (2005), Seiten 145 bis 151, ein Verfahren mit den Merkmalen des Oberbegriffes des Patentanspruches 1 bekannt.

Für den routinemäßigen Gebrauch in Krankenhäusern, Pflegeeinrichtungen sowie Arztpraxen sind diese analytischen Verfahren jedoch nach wie vor nur bedingt geeignet, da eine zuverlässige Messung nicht gewährleistet ist und es vielfach zur Messung des Hintergrundes (Reinigungsmittel, sonstige Verunreinigungen) bzw. einer falschen Zuordnung der Messergebnisse kommt. Auch eluieren verschiedene flüchtige Verbindungen (z.B. Ketone) und sonstige Komponenten aufgrund ihrer Adsorption an den Leitungen des Messsystems vielfach erst in Folgemessungen von der gaschromatografischen Säule und verfälschen die tatsächlichen Ergebnisse.

Die Ergebnisse dieses ansonsten vielversprechenden Verfahrens können insofern den Anforderungen eines Routineeinsatzes noch nicht gerecht werden, so dass die Gaschromatografie-Ionenmobilitätsspektrometrie, deren Vorteile in ihrer Empfindlichkeit liegen, Probleme hinsichtlich ihrer wissenschaftlichen Akzeptanz hat.

Aus DE 10 2007 033 906 A1 ist ein Verfahren zur Messung der menschlichen Ausatemluft mittels Gaschromatografie-Ionenmobilitätsspektrometrie bekannt, bei welchem eine Ausatemluftprobe über einen Probeneinlass und ein Mehrwegeventil in eine Probenschleife gelangt und nachfolgend mittels eines Trägergases aus der Probenschleife über das Mehrwegeventil durch eine gaschromatorafische Säule in einen Ionenmobilitätsspektrometer gefördert und gemessen wird. Ferner ist ein Spülgaseinlass vorgesehen.

Aufgabe der Erfindung ist es, ein Verfahren zur Messung der menschlichen Ausatemluft mittels Gaschromatografie-Ionenmobilitätsspektrometrie anzugeben, welches zuverlässige und korrekte Messergebnisse liefert.

Diese Aufgabe wird bei einem Verfahren der eingangs bezeichneten Art erfindungsgemäß dadurch gelöst, dass vor der Zuführung einer Ausatemluftprobe in die Probenschleife
a) zunächst wenigstens die gaschromatografische Säule, das Ionenmobilitätsspektrometer und die Probenschleife mit einem Spülgas durchspült werden und das Mehrwegeventil anschließend so geschaltet wird, dass das Spülgas in das Ionenmobilitätsspektrometer gelangt und gemessen wird,
b) danach die Spülgaszufuhr beendet und das Mehrwegeventil so geschaltet wird, dass Umgebungsluft durch die gaschromatografische Säule in das Ionenmobilitätsspektrometer gelangt und gemessen wird,
c) anschließend wenigstens die gaschromatografische Säule, das Ionenmobilitätsspektrometer und die Probenschleife mit angefeuchtetem Spülgas durchspült werden und das Mehrwegeventil so geschaltet wird, dass das angefeuchtete Spülgas in das Ionenmobilitätsspektrometer gelangt und gemessen wird,
d) die Zufuhr des angefeuchteten Spülgases beendet und eine Ausatemluftprobe in die Probenschleife geleitet und mittels des Trägergases durch die gaschromatografische Säule in das Ionenmobilitätsspektrometer gefördert und gemessen wird.

Das erfindungsgemäße Verfahren ist somit derart gestaltet, dass Kontaminationen vor einer tatsächlichen Messung und Fehlmessungen zuverlässig vermieden werden, indem vor der eigentlichen Atemluftmessung zunächst nacheinander die vorbeschriebenen Schritte durchgeführt werden, d.h. in einem ersten Verfahrensschritt eine Freimessung der Sensoren (gaschromatografische Säule und Ionenmobilitätsspektrometer) ohne Probenmessung, in einem zweiten Schritt die Umgebungsluft und in einem dritten Schritt eine Freimessung des Systems mit angefeuchtetem Spülgas als Probe durchgeführt wird, so dass nach Entfernung von Kontaminationen durch den Spülvorgang die Messsystemsystemparameter sowie die Umgebungsbedingungen berücksichtigt werden, d.h. sowohl die Messsystemparameter (z.B. Reaktionsionenpeak (RIP)) als auch die Zusammensetzung bzw. Beschaffenheit der Umgebungsluft und der Feuchtegehalt erfasst werden, so dass diese erfassten Parameter bei der nachfolgenden Messung und Auswertung der Ausatemluftprobe berücksichtigt werden.

Um nach Abschluss der Messung eine Atemluftprobekontamination zu vermeiden, ist bevorzugt vorgesehen, dass nach Messung einer Atemluftprobe wenigstens die gaschromatographische Säule, das Ionenmobilitätsspektrometer, die Probenschleife und der Probeneinlass mit einem Spülgas durchspült werden. Diese Durchspülung wird solange aufrechterhalten, bis ein neuer Atemluftmessvorgang erfolgen soll bzw. die Messeinrichtung abgeschaltet wird.

In besonders bevorzugter Ausgestaltung ist vorgesehen, dass als Probeneinlass ein Spirometer verwendet wird. Zur validierbaren und reproduzierbaren Probennahme wird bevorzugt ein medizinisches Spirometer eingesetzt, dessen Sensoren zur präzisen und unmittelbaren Datenaufnahme von CO2/O2 und Volumenstrom im Handgehäuse integriert sind. Die Übergangsschläuche zur eigentlichen Messeinrichtung werden außerhalb eines Messvorganges mit Spülgas durchflutet und sind bevorzugt beheizt, um Kondensationen zu vermeiden und Kontaminationen reinigen zu können.

Dabei ist bevorzugt vorgesehen, dass die Durchflussmenge der Ausatemluft durch das Spirometer überprüft und bei Unterschreiten eines vorgegebenen Grenzwertes die Ausatemluftzufuhr in die Probenschleife unterbrochen wird. Wenn z.B. ein Patient nicht in der Lage ist, ausreichend Atemluft in das Spirometer zu blasen, wird die Ausatemluftzufuhr unterbrochen, um zu vermeiden, dass Umgebungsluft in das Messsystem gelangt. Als Grenzwert bei vorgegebenen Durchflussbedingungen des Spirometers kann z.B. ein Zeitintervall eingerichtet sein. So kann z.B. vorgesehen sein, dass nur mehrere Sekunden fortwährend in das Mundstück des Spirometers ausgeatmet werden muss. Wird der Ausatemvorgang unterbrochen bzw. vorzeitig beendet, wird die Ausatemluftzufuhr unterbrochen.

Alternativ kann vorgesehen sein, dass die Durchflussmenge der Ausatemluft durch das Spirometer überprüft und erst nach Überschreiten eines vorgegebenen Grenzwertes die Ausatemluftzufuhr in die Probenschleife freigegeben wird. Ein Patient kann dann mehrmals kleinere Volumina ausatmen, die dann addiert werden, so dass ein ausreichend großes Probenvolumen (Atemluft) zur Verfügung steht, welches in die Probenschleife gelangt.

Ferner ist bevorzugt vorgesehen, dass ein Kalibrier- oder Testgas oder eine externe Atemluftprobe aus einem Probengefäß über einen zusätzlichen Gaseinlass dem Mehrwegeventil zugeführt wird.

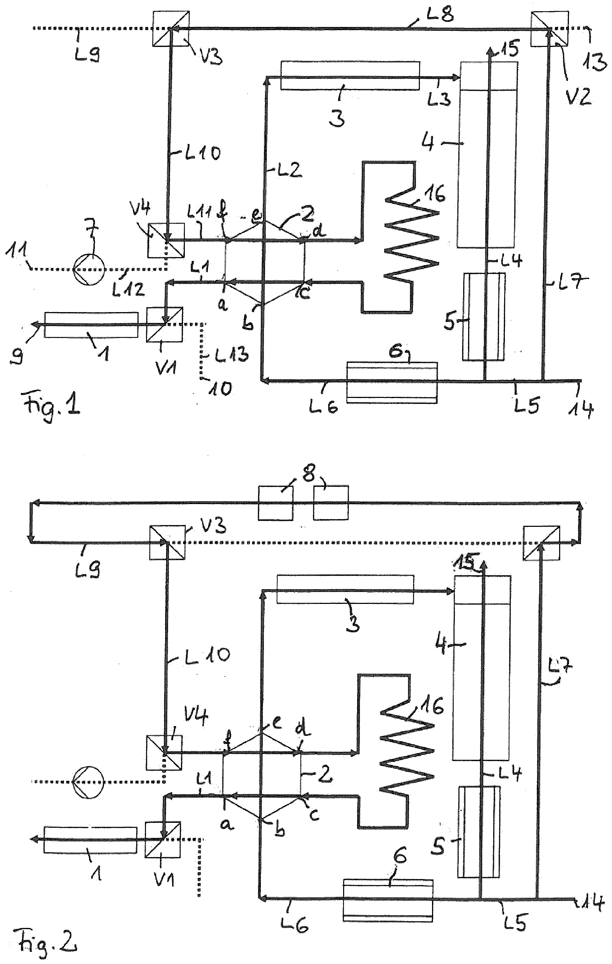

Die Erfindung ist nachstehend anhand der Zeichnung beispielhaft näher erläutert. Diese zeigt in den
- Fig. 1 bis 6: in schematischer Darstellung eine Messeinrichtung zur Messung der menschlichen Ausatemluft mittels Gaschromatografie-Ionen-mobilitätsspektrometrie in verschiedenen Messablaufstadien.

Die Messeinrichtung weist zunächst ein Spirometer 1 auf, welches über ein Umschaltventil V1 und eine Leitung L1 mit einem Mehrwegeventil, beim Ausführungsbeispiel einem 6-Wege-Ventil 2 in Verbindung steht. Die sechs Ein- bzw. Ausgänge des 6-Wege-Ventils 2 sind mit a, b, c, d, e, f bezeichnet. An den Ein- bzw. Ausgang c, d des 6-Wege-Ventils 2 ist eine Probenschleife 16 angeschlossen. Der Ausgang e des 6-Wege-Ventils 2 steht über eine Leitung L2 mit einer gaschromatografischen Säule 3, vorzugsweise einer Multikapillarsäule, in Fluidverbindung, deren Ausgang über eine Leitung L3 mit dem Ionisatorraum eines Ionenmobilitätsspektrometers 4 verbunden ist. An den Driftgaseingang des Ionenmobilitätsspektrometers 4 ist eine Leitung L4 angeschlossen, welcher mit einer elektronischen Druckregelung 5 für das Driftgas ausgerüstet ist. Die Leitung L4 steht als Abzweigleitung mit einer Gaszuführleitung L5 in Fluidverbindung, die endseitig mit einem Gaseinlass 14 verbunden ist. Von der Leitung L5 zweigt darüber hinaus eine Leitung L6 ab, welche mit einer elektronischen Druckregelung 6 für ein Trägergas ausgerüstet ist. Die Leitung L6 endet am Einlass b des 6-Wege-Ventils 2.

Beim Ausführungsbeispiel ist somit nur ein Gaseinlass 14 für das Trägergas und das Driftgas vorgesehen, d.h. diese sind beim Ausführungsbeispiel identisch, z.B. Stickstoff oder synthetische Luft. Ferner zweigt von der Leitung L5 eine Leitung L7 ab, die über ein Umschaltventil V2 mit einem Gasauslass 13 oder einer Leitung L8 in Verbindung stehen kann. Die Leitung L8 steht über ein weiteres Umschaltventil V3 mit einer Leitung L9 oder einer Leitung L10 in Verbindung. Die Leitung L10 steht über ein Umschaltventil V4 entweder mit einer Leitung L11, die an den Anschluss f des 6-Wege-Ventils 2 angeschlossen ist, oder mit einer Leitung L12 in Verbindung, in welcher eine Pumpe 7 angeordnet ist und die in einem Probenausgang 11 endet. Die Leitung L9 ist, wie aus Figur 2 hervorgeht, an externe Wasserflaschen 8 an-geschlossen und mündet am Umschaltventil V2.

Ein Probeneingang am Spirometer 1 ist mit 9 bezeichnet, an das Umschaltventil V1 des Spirometers 1 ist über eine Leitung L13 ein Kalibrationseingang 10 angeschlossen. Ferner ist der Gasauslass des Ionenmobilitätsspektrometers 4 mit 15 bezeichnet.

In den Figuren 1 bis 6 sind verschiedene Verfahrensablaufstadien dargestellt. Je nach Ventilstellung des 6-Wege-Ventils 2 und der weiteren Ventile V1, V2, V3, V4 sind jeweils nur einzelne Leitungen freigegeben, die freigegebenen, d.h. aktiven Leitungen sind in den Figuren 1 bis 6 als durchgezogene Linien dargestellt. Dagegen sind nicht aktive Leitungen als punktierte Linien dargestellt.

Das über den Gaseinlass 14 zuführbare Driftgas zum Spülen und Erzielen optimaler Ergebnisse des Ionenmobilitätsspektrometers 4 wird durch die elektronische Druckregelung 5 kontrolliert. Das Probenträgergas, welches über die gaschromatografische Säule 3 und anschließend in das Ionenmobilitätsspektrometer 4 geleitet wird, wird von der elektronischen Druckregelung 6 gesteuert. Beide Gase (Drift- und Probenträgergas), z.B. Stickstoff oder synthetische Luft, werden auf separaten Wegen zum Gasauslass 15 geführt. Sowohl das Ionenmobilitätsspektrometer 4 als auch die gaschromatografische Säule 3 sowie das 6-Wege-Ventil 2 sind vorzugsweise temperaturgesteuert.

Solange keine Messung der Atemluft oder auch eines Test-/Kalibriergases durchgeführt wird, wird das Messsystem mit Spülgas gespült. Zur Sauberkeit des Gesamtsystems spült das Spülgas zusätzlich auch durch das Spirometer 1, um Adsorptionen von Substanzen von vorangehenden Messungen an den internen Leitungen L1, L7, L8, L10 und L11, den Ventilen V1 und V4, der Probenschleife 16 und den Anschlüssen a, b, c, d, e, f des 6-Wege-Ventils 2 zu verhindern.

Eine Gasprobe wird mittels der Pumpe 7 in das System gesaugt. Die Probennahme der Atemluft kann direkt durch Ausatmen in ein in einen Halter des Spirometers 1 gestecktes auswechselbares Mundstück erfolgen. Über die vorzugsweise beheizte Leitung L1 wird die Probe zum 6-Wege-Ventil 2 transportiert. Alternativ und zu Kalibrierzwecken kann die Probe auch von einer Gasflasche oder einem Gasprobenbehältnis über den Kalibriereingang 10 in die Leitung L13 gegeben werden.

Zur Messung einer Gasprobe aus der Atemluft oder einer Testgasquelle spült in der Grundeinstellung des 6-Wege-Ventils 2, welche in Figur 1 dargestellt ist, Trägergas permanent durch die gaschromatografische Säule 3. Ein Probengas wird über das Spirometer 9 oder über den Kalibriereingang 10 mittels der Pumpe 7 durch die Probenschleife 16 gesaugt. In dieser Position wird das Probengas vom Spirometer 1 oder Kalibrationseingang 10 direkt zum Gasauslass 11 geführt.

Zur Durchführung der eigentlichen Messung wird die Probe in der Probenschleife 16 zur gaschromatografischen Säule 3 und nachfolgend zum Ionenmobilitätsspektrometer 4 durch Schalten des 6-Wege-Ventils 2 transportiert. Das Trägergas befördert so die Atemluftprobe in die Probenschleife 16 und weiter zur gaschromatografischen Säule 3, wo die sich in der Probe befindlichen Substanzen ihrer Retentionszeit entsprechend getrennt werden. Die eluierenden Substanzen werden über die Leitung L3 in den Ionisationsraum des Ionenmobilitätsspektrometers 4 eingeleitet.

Zur validierbaren und reproduzierbaren Probennahme wird ein medizinisches Spirometer 1 eingesetzt, dessen Sensoren zur präziseren und unmittelbaren Datenaufnahme von CO2/O2 und Volumenstrom im Handgehäuse integriert sind. Die Anschlussleitung L1 wird in der Grundeinstellung mit Spülgas durchflutet und ist beheizt, um Kondensationen zu vermeiden, und Kontaminationen reinigen zu können. Der Zeitpunkt einer Schaltung des 6-Wege-Ventils 2 und damit der Probennahme kann gemäß analytischer Fragestellung mittels CO2/O2 bzw. Volumenstrommessung der Atemluft durch Kommunikation zwischen dem Spirometer 1 und der Steuerung der Messeinrichtung variiert/optimiert und im Programmablauf gespeichert werden.

Die verschiedenen Systemeinstellungen des Messsystems und damit der Messverfahrensablauf sind wie folgt:
In Figur 1 ist die Grundeinstellung dargestellt, dass Spülgas (Drift- sowie Probenträgergas) strömt vom Gaseinlass 14 über die aktiven Leitungen (durchgezogene Linien) einerseits als Driftgas durch das Ionenmobilitätsspektrometer 4, andererseits über die entsprechend geschalteten Ein- bzw. Ausgänge b, e des 6-Wege-Ventils 2 als Probengas durch die gaschromatografische Säule 3 und das Ionenmobilitätsspektrometer 4 und ferner über die miteinander geschalteten Anschlüsse f, d, c und a des 6-Wege-Ventils 2 durch die Probenschleife 16 und das Spirometer 1. In dieser Grundeinstellung werden somit sämtliche Systemkomponenten vom Spülgas durchströmt.

Zu Beginn einer Atemluftmessung wird in dieser Grundeinstellung gemäß Figur 1 eine Freimessung des Systems durchgeführt, d.h. während der Durchspülung der Systemkomponenten gelangt sozusagen als Probengas das Spülgas in den Ionisationsraum des Ionenmobilitätsspektrometers 4 und das Spülgas wird im Ionenmobilitätsspektrometer 4 gemessen.

Die gemessenen Werte werden entsprechend in der Systemsteuerung gespeichert und bei der späteren Probenmessung bzw. Messungsauswertung berücksichtigt.

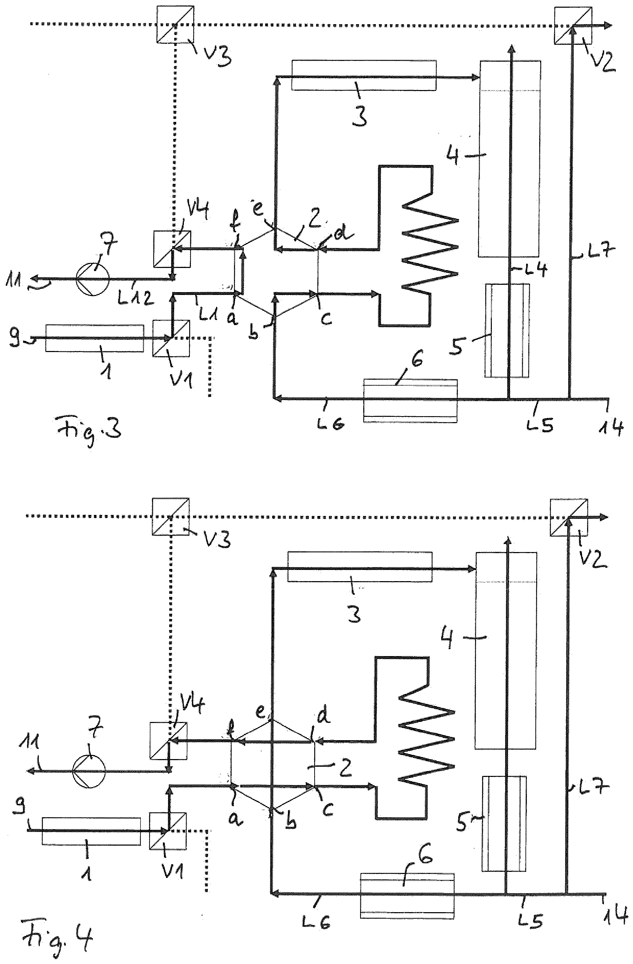

In einem zweiten Verfahrensschritt wird die Spülgaszufuhr beendet und das Mehrwegeventil 2 so geschaltet, dass Umgebungsluft durch die gaschromatografische Säule 3 in das Ionenmobilitätsspektrometer 4 gelangt und dort gemessen wird. Dazu saugt die Pumpe 7 Umgebungsluft durch das Spirometer 1 zur gasdichten Probenschleife 16. Das Mehrwegeventil 2 befindet sich dann in der in Figur 4 dargestellten Schaltposition. Anschließend wird das 6-Wege-Ventil in die in Figur 3 dargestellte Position geschaltet, so dass eine Probe, in diesem Fall Umgebungsluft, zur gaschromatografischen Säule 3 und weiter zum Ionenmobilitätsspektrometer transportiert wird und die Messungsdaten aufgenommen werden. Die Messdaten der Umgebungsluft werden entsprechend verarbeitet.

In einem dritten Verfahrensschritt werden anschließend wenigstens die gaschromatografische Säule 3 des Ionenmobilitätsspektrometers 4 und die Probenschleife 16 mit angefeuchtetem Spülgas durchspült. Diese Situation ist in Figur 2 dargestellt, das über den Gaseinlass 14 eintretende Spülgas wird über die Leitung L8 durch externe Wasserflaschen 8 geführt und befeuchtet und gelangt somit auch in die Probenschleife 16. Anschließend wird das 6-Wege-Ventil 2 in die in Figur 3 dargestellte Position geschaltet, so dass die Probe, in diesem Fall z.B. befeuchtetes N2 oder synthetische Luft weiter zur gaschromatografischen Säule 3 und zum Ionenmobilitätsspektrometer 4 transportiert wird und die Messungsdaten aufgenommen werden. Auch die Messungsdaten dieses dritten Verfahrensschrittes werden gespeichert und entsprechend bei der späteren Auswertung der Atemluftprobe berücksichtigt.

Anschließend wird die Spülgaszufuhr beendet und im letzten Schritt eine Ausatemluftprobe eines Patienten in die Probenschleife 16 geleitet. In der in Figur 4 dargestellten Schaltposition des 6-Wege-Ventils 2 gelangt die Atemluftprobe vom Spirometer 1 in die Probenschleife 16. Die Atemluftprobe wird dabei von der Pumpe 7 angesaugt.

Dabei wird der Patient softwaretechnisch aufgefordert, kontinuierlich in das Mundstück des Spirometers 1 zu atmen, um die Probenschleife 16 zu füllen. Es ist z.B. erforderlich, für 6 Sekunden fortwährend in das Mundstück des Spirometers 1 zu atmen. Sollte der Patient nicht in der Lage sein, die vorgegeben Ausatmungsprozedur durchzuführen und/oder diese während der vorgegebenen Zeitdauer unterbrechen, wird das Ventil V1 zurückgeschaltet und die Pumpensteuerung der Pumpe 7 unterbricht den Saugprozess. Dies verhindert, dass Umgebungsluft in das System gelangt.

Wenn hingegen die Ausatemluft korrekt in die Probenschleife 16 gelangt, wird nach Befüllung derselben das Mehrwegeventil 2 in die Position gemäß Figur 3 geschaltet und die Atemluftprobe wird vom Trägergas durch die gaschromatografische Säule 3 in das Ionenmobilitätsspektrometer 4 gefördert und dort gemessen. Anschließend erfolgt dann die Messwertauswertung der Atemluftprobe unter Berücksichtigung der vorausgehenden Messungen.

Ist ein Patient nicht in der Lage, für z.B. sechs Sekunden fortwährend in das Mundstück des Spirometers 1 zu atmen, besteht softwaretechnisch die Möglichkeit, die einzelnen kleineren Volumina zu addieren, in dem nach jeder Ausatemprozedur das Ventil V1 zurückschaltet und erst wenn ein ausreichend großes Gesamtvolumen zur Verfügung steht, wird das 6-Wege-Ventil 2 geschaltet und die Probe gelangt aus der Probenschleife 16 in die Säule 3 und nachfolgend in das Ionenmobilitätsspektrometer 4.

Nach Beendigung der Zufuhr der Atemluftprobe erfolgt eine Umschaltung in die Grundeinstellung gemäß Figur 1, d.h. die Systemkomponenten werden mit Spülgas durchströmt, bevor ein neuer Messzyklus beginnt bzw. die Messeinrichtung ausgeschaltet wird.

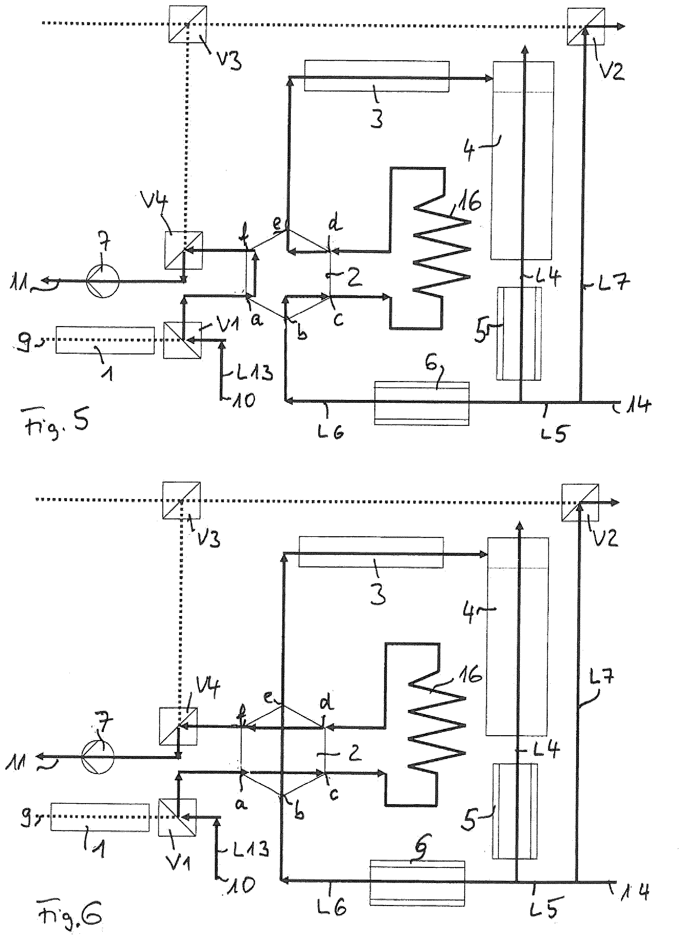

In den Figuren 5 und 6 ist eine zusätzliche Verfahrensführung gezeigt, die zur Messung von Test-/Kalibriergas oder Proben aus externen Probenbehältnissen bei Zuführung über den Kalibriereingang 10 dient. Ausgehend von der Grundeinstellung gemäß Figur 1 wird die Spülgaszuführung beendet und das Ventil V1 zwischen dem Spirometer 1 und dem Kalibriereingang 10 wird vom Spirometer 1 auf den Kalibriereingang 10 geschaltet und der Gasfluss wird umgeleitet. Dabei befindet sich das Mehrwegeventil 2 zunächst in der Position gemäß Figur 6. Dabei saugt die Pumpe 7 aus dem Kalibriereingang 10 in die Probenschleife 16. Anschließend wird das 6-Wege-Ventil in die in Figur 5 dargestellte Position geschaltet, so dass die Probe, in diesem Fall Test- oder Kalibriergas bzw. Probengas, aus einem externen Behältnis zur gaschromatografischen Säule 5 und weiter zum Ionenmobilitätsspektrometer 4 transportiert wird und die Messungsdaten aufgenommen werden. Nach Beendigung dieses zusätzlichen Verfahrensschrittes wird das Messsystem wieder in seine Grundstellung (Spülmodus) gemäß Figur 1 zurückgesetzt.

## Patentansprüche

1. Verfahren zur Messung der menschlichen Ausatemluft mittels Gaschromatografie-Ionenmobilitätsspektrometrie, bei welchem eine Ausatemluftprobe über einen Probeneinlass (9) und ein Mehrwegeventil (2) in eine Probenschleife (16) gelangt und nachfolgend mittels eines Trägergases aus der Probenschleife über das Mehrwegeventil durch eine gaschromatographische Säule (3) in ein Ionenmobilitätsspektrometer (4) gefördert und gemessen wird, **dadurch gekennzeichnet,**
**dass** vor der Zuführung einer Ausatemluftprobe in die Probenschleife
a) zunächst wenigstens die gaschromatografische Säule, das Ionenmobilitätsspektrometer und die Probenschleife mit einem Spülgas durchspült werden und das Mehrwegeventil anschließend so geschaltet wird, dass das Spülgas (14) in das Ionenmobilitätsspektrometer gelangt und gemessen wird,
b) danach die Spülgaszufuhr beendet und das Mehrwegeventil so geschaltet wird, dass Umgebungsluft durch die gaschromatografische Säule in das Ionenmobilitätsspektrometer gelangt und gemessen wird,
c) anschließend wenigstens die gaschromatografische Säule, das Ionenmobilitätsspektrometer und die Probenschleife mit angefeuchtetem Spülgas durchspült werden und das Mehrwegeventil so geschaltet wird, dass das angefeuchtete Spülgas in das Ionenmobilitätsspektrometer gelangt und gemessen wird,
d) die Zufuhr des angefeuchteten Spülgases beendet und eine Ausatemluftprobe in die Probenschleife geleitet und mittels des Trägergases durch die gaschromatographische Säule in das Ionenmobilitätsspektrometer gefördert und gemessen wird.

2. Verfahren nach Anspruch 1,
**dadurch gekennzeichnet,**
**dass** nach Messung einer Atemluftprobe wenigstens die gaschromatografische Säule, das Ionenmobilitätsspektrometer, die Probenschleife und der Probeneinlass mit einem Spülgas durchspült werden.

3. Verfahren nach Anspruch 1 oder 2,
**dadurch gekennzeichnet,**
**dass** als Probeneinlass ein Spirometer verwendet wird.

4. Verfahren nach Anspruch 3,
**dadurch gekennzeichnet,**
**dass** die Durchflussmenge der Ausatemluft durch das Spirometer überprüft und bei Unterschreiten eines vorgegebenen Grenzwertes die Ausatemluftzufuhr in die Probenschleife unterbrochen wird.

5. Verfahren nach Anspruch 3,
**dadurch gekennzeichnet,**
**dass** die Durchflussmenge der Ausatemluft durch das Spirometer überprüft und erst nach Überschreiten eines vorgegebenen Grenzwertes die Ausatemluftzufuhr in die Probenschleife freigegeben wird.

6. Verfahren nach einem oder mehreren der Ansprüche 1 bis 5,
**dadurch gekennzeichnet,**
**dass** ein Kalibrier- oder Testgas oder eine externe Atemluftprobe aus einem Probengefäß über einen zusätzlichen Gaseinlass dem Mehrwegeventil zugeführt wird.

## Claims

1. Method for measuring human exhaled air by means of gas chromatography/ion mobility spectrometry, in which an exhaled-air sample is passed via a sample inlet (9) and a multi-way valve (2) into a sample loop (16) and subsequently is conveyed by means of a carrier gas out of the sample loop via the multi-way valve through a gas-chromatography column (3) into an ion-mobility spectrometer (4) and measured,
**characterised in that**
before the feed of an exhaled-air sample into the sample loop
a) firstly, at least the gas-chromatography column, the ion-mobility spectrometer and the sample loop are rinsed with a purge gas (14) and the multi-way valve is then switched such that the purge gas is passed into the ion-mobility spectrometer and measured,
b) thereafter the purge-gas feed is ended and the multi-way valve is switched such that ambient air is passed through the gas-chromatography column into the ion-mobility spectrometer and measured,
c) then at least the gas-chromatography column, the ion-mobility spectrometer and the sample loop are rinsed with humidified purge gas and the multi-way valve is switched such that the humidified purge gas is passed into the ion-mobility spectrometer and measured,
d) the feed of the humidified purge gas is ended and an exhaled-air sample is injected into the sample loop and conveyed by means of the carrier gas through the gas-chromatography column into the ion-mobility spectrometer and measured.

2. Method according to claim 1,
**characterised in that**
after measuring a breath sample, at least the gas-chromatography column, the ion-mobility spectrometer, the sample loop and the sample inlet are rinsed with a purge gas.

3. Method according to claim 1 or 2,
**characterised in that**
a spirometer is used as sample inlet.

4. Method according to claim 3,
**characterised in that**
the flow rate of the exhaled air is monitored by the spirometer and in case of a drop below a predetermined limit value the exhaled-air feed into the sample loop is interrupted.

5. Method according to claim 3,
**characterised in that**
the flow rate of the exhaled air is monitored by the spirometer and the exhaled-air feed into the sample loop is released only after a predetermined limit value is exceeded.

6. Method according to one or more of claims 1 to 5,
**characterised in that**
a calibration or test gas or an external breath sample is fed from a sampling vessel via an additional gas inlet to the multi-way valve.

## Revendications

1. Procédé de mesure de l'air expiré chez l'homme au moyen de la chromatographie en phase gazeuse-spectrométrie de mobilité ionique, dans lequel un échantillon d'air expiré parvient par une entrée d'échantillon (9) et une vanne multivoie (2) dans une boucle d'échantillon (16) et passe ensuite, au moyen d'un gaz vecteur, de la boucle d'échantillon par la vanne multivoie via une colonne de chromatographie en phase gazeuse (3) dans un spectromètre de mobilité ionique (4) et est mesuré, **caractérisé en ce qu'**
avant d'amener un échantillon d'air expiré dans la boucle d'échantillon
a) au moins la colonne de chromatographie en phase gazeuse, le spectromètre de mobilité ionique et la boucle d'échantillon sont d'abord purgés avec un gaz de purge (14)
et la vanne multivoie est ensuite actionnée de sorte que le gaz de lavage parvient dans le spectromètre de mobilité ionique et est mesuré,
b) puis, l'arrivée de gaz de lavage est stoppée et la vanne multivoie est actionnée de sorte que l'air ambiant parvient via la colonne de chromatographie en phase gazeuse dans le spectromètre de mobilité ionique et est mesuré,
c) ensuite, au moins la colonne de chromatographie en phase gazeuse, le spectromètre de mobilité ionique et la boucle d'échantillon sont purgés avec du gaz de purge humidifié et la vanne multivoie est actionnée de sorte que le gaz de purge humidifié parvient dans le spectromètre de mobilité ionique et est mesuré,
d) l'apport en gaz de purge humidifié est stoppé et un échantillon d'air expiré est conduit dans la boucle d'échantillon et fait passer au moyen du gaz vecteur via la colonne de chromatographie en phase gazeuse dans le spectromètre de mobilité ionique et est mesuré.

2. Procédé selon la revendication 1,
**caractérisé en ce qu'**
après avoir mesuré un échantillon d'air, au moins la colonne de chromatographie en phase gazeuse, le spectromètre de mobilité ionique, la boucle d'échantillon et l'entrée d'échantillon sont purgés avec un gaz de purge.

3. Procédé selon la revendication 1 ou 2,
**caractérisé en ce qu'**
un spiromètre est utilisé en tant qu'entrée d'échantillon.

4. Procédé selon la revendication 3,
**caractérisé en ce que**
le débit de l'air expiré est examiné via le spiromètre et l'apport en air expiré dans la boucle d'échantillon est interrompu dans le cas où il est en dessous d'une valeur limite prédéterminée.

5. Procédé selon la revendication 3,
**caractérisé en ce que**
le débit de l'air expiré est examiné via le spiromètre et l'apport en air expiré dans la boucle d'échantillon est seulement libéré après le dépassement d'une valeur limite prédéterminée.

6. Procédé selon l'une quelconque ou plusieurs des revendications 1 à 5,
**caractérisé en ce que**
le gaz de calibrage ou de test ou un échantillon d'air externe d'un récipient pour échantillon est amené à la vanne multivoie par une entrée de gaz supplémentaire.
